# EUROPEAN PATENT SPECIFICATION

(11) **EP 2 073 841 B1**
(45) Date of publication and mention of the grant of the patent: **26.11.2014**
(21) Application number: 07825522.1
(22) Date of filing: 15.08.2007
(51) Int. Cl.: A61K 39/295, A61K 39/10, A61K 39/05, A61K 39/13, A61K 39/08, A61K 39/29

(54) **MANUFACTURE OF BOOSTER VACCINES HAVING REDUCED ANTIGEN DOSES**
HERSTELLUNG VON BOOSTER-IMPFSTOFFEN MIT VERRINGERTEN ANTIGENDOSEN
FABRICATION DE VACCINS AMPLIFICATEURS À DOSES RÉDUITES D'ANTIGÈNE

(30) Priority: 16.08.2006 GB 0616306
(43) Date of publication of application: 01.07.2009
(73) Proprietor: GlaxoSmithKline Biologicals S.A., 1330 Rixensart (BE)
(72) Inventor: DE HEYDER, Koenraad, Rixensart B-1330 (BE); ARTOIS, Claude, Rixensart B-1330 (BE); VANDECASSERIE, Christian, Rixensart B-1330 (BE)
(74) Representative: Lubienski, Michael John
(86) International application number: PCT/IB2007/003255
(87) International publication number: WO 2008/020328

(56) References cited:
- EP-A- 1 004 314
- WO-A-93/24148
- WO-A-98/19702
- WO-A-99/13906
- WO-A-02/055105
- LAROCHE P ET AL: "THE IMMUNOGENICITY AND SAFETY OF A NEW COMBINED DIPHTHERIA, TETANUS AND POLIOMYELITIS BOOSTER VACCINE (TD-EIPV)" INFECTION, MMV MEDIZIN VERLAG, MUENCHEN, DE, vol. 27, no. 1, January 1999 (1999-01), pages 49-56, XP000891892 ISSN: 0300-8126
- JONES TAFF: "Boostrix (GlaxoSmithKline)." IDRUGS : THE INVESTIGATIONAL DRUGS JOURNAL AUG 2005, vol. 8, no. 8, August 2005 (2005-08), pages 656-661, XP002474713 ISSN: 1369-7056
- AMERICA ACADEMY OF PEDIATRICS COMMITEE ON INFECTIOUS DISEASES: "Prevention of pertussis among adolescents: recommendations for use of tetanus toxoid, reduced diphtheria toxoid, and acellular pertussis (Tdap) vaccine." PEDIATRICS MAR 2006, vol. 117, no. 3, March 2006 (2006-03), pages 965-978, XP002474714 ISSN: 1098-4275
- GRIMPREL E ET AL: "Combined reduced-antigen-content diphtheria-tetanus-acellular pertussis and polio vaccine (dTpa-IPV) for booster vaccination of adults" VACCINE, BUTTERWORTH SCIENTIFIC. GUILDFORD, GB, vol. 23, no. 28, 25 May 2005 (2005-05-25), pages 3657-3667, XP004887025 ISSN: 0264-410X

## Description

### TECHNICAL FIELD

This invention is in the field of manufacturing combination vaccines, that is vaccines containing mixed immunogens from more than one pathogen, such that administration of the vaccine can simultaneously immunize a subject against more than one pathogen.

### BACKGROUND ART

In 2005, two adolescent booster vaccines were licensed in the USA - BOOSTRA™ and ADACEL™ [1]. Both vaccines contain diphtheria toxoid, tetanus toxoid and acellular pertussis antigens. A common feature of these two vaccines is that, relative to their pediatric counterparts, they have lower antigen doses e.g. the diphtheria toxoid content of BOOSTRIX™ is 10-fold lower than in the INFANRIX™ products, and in ADACEL™ it is 7.5-fold lower than in DAPTACEL™. Moreover, the ratio of antigenic components is also altered. In particular, the ratio of diphtheria and tetanus toxoids is 2.5:1 in the INFANRIX™ products but is 1:2 in the BOOSTRIX™ product, and is 3:1 in the DAPTACEL™ product but is 1:2.5 in the ADACEL™ product.

Thus these booster vaccines show a large reduction in the dose of diphtheria toxoid, both in absolute amounts and also relative to the tetanus toxoid content.

It is an object of the invention to provide further and improved methods for manufacturing vaccine compositions of this type.

### DETAILED DESCRIPTION OF THE INVENTION

Rather than manufacture booster vaccine compositions by a process involving mixing individual bulk Diphtheria and tetanus antigens, the invention uses (i) a first bulk comprising both diphtheria toxoid and tetanus toxoid, and (ii) a second bulk comprising tetanus toxoid without diphtheria toxoid. This arrangement facilitates convenient manufacture of both pediatric and adolescent vaccines from the same bulks: the first bulk can have a diphtheria:tetanus ratio that is suitable for pediatric vaccines, and the second bulk can be used to reduce the relative amount of diphtheria toxoid, as found in the adolescent vaccines.

Thus a single bulk can fulfill two roles for a vaccine manufacturer, which can simplify matters. For instance, a mixed diphtheria/tetanus bulk can be prepared and tested for clinical release parameters (sterility, potency, antigen content, *etc.*) and then kept in storage. If a pediatric vaccine is required then the bulk can be used without further adjustment of the diphtheria/tetanus ratio, but if an adolescent vaccine is required then no further diphtheria materials have to be prepared. Monovalent diphtheria toxoid bulk does not have to be prepared or tested or stored, thereby simplifying the manufacturing process. With a mixed diphtheria/tetanus bulk in stock, a manufacturer can readily prepare pediatric vaccines; by additionally stocking monovalent tetanus toxoid bulk to supplement the diphtheria/tetanus bulk, a manufacturer can easily swap between pediatric and adolescent formulations without requiring a separate monovalent diphtheria toxoid bulk e.g. depending on changes in demand from healthcare providers.

The invention therefore provides a process for manufacturing a vaccine, wherein the vaccine comprises diphtheria toxoid and tetanus toxoid and, and wherein the process comprises steps of combining (i) a first bulk comprising diphtheria toxoid and tetanus toxoid with (ii) a second bulk comprising tetanus toxoid but no diphtheria toxoid.

The vaccine may comprise further antigens, such as acellular pertussis antigens, inactivated poliovirus antigens, *etc*. These further antigens may be used in the process as separate bulks, and/or they may be present in the first and/or second bulk at the start of the process.

The vaccine may comprise an adjuvant, such as an adjuvant comprising one or more aluminium salts *e.g.* aluminium hydroxide. Adjuvants may be used in the process as separate bulks, and/or they may be present in the first and/or second bulk before the start of the process.

### The first aqueous bulk

The first bulk used in the processes of the invention comprises both diphtheria toxoid and tetanus toxoid.

The diphtheria and tetanus toxoids in the first bulk are preferably adsorbed (more preferably totally adsorbed) onto an aluminum hydroxide adjuvant. Adsorption can be achieved by preparing the toxoids separately, adsorbing each of them separately to an aluminum hydroxide adjuvant, and then mixing the two adsorbed toxoids (optionally with further adjuvant and any other desired components) to give the first bulk.

The first bulk may include a preservative, such as 1-hydroxy-2-phenoxyethane (also known as '2-hydroxyethyl phenyl ether', '2-phenoxyethanol', 'ethyleneglycol phenyl ether', *etc*.). Preferred bulks, however, are free from preservatives, and in particular are free from 1-hydroxy-2-phenoxyethane. If the first bulk does contain 1-hydroxy-2-phenoxyethane, however, its concentration is preferably (a) between 2.5 mg and 3.5 mg (*e.g.* about 3 mg) for every 100 Lf of diphtheria toxoid, and/or (b) between 7 mg and 8 mg (*e.g.* about 7.5 mg) for every 100 Lf of tetanus toxoid. A 1-hydroxy-2-phenoxyethane concentration of between 3 g/l and 8 g/l (*e.g.* between 4-6 g/l, or about 5 g/l) may be used in the first bulk. The first bulk is free from mercurial preservatives (*e.g.* thimerosal).

The ratio (measured in Lf units) of diphtheria toxoid to tetanus toxoid in the first component is between 2:1 and 3:1, and is preferably about 2.5:1.

The concentration of diphtheria toxoid and tetanus toxoid in first bulk may be as follows, expressed per milliliter (±10%): 167 Lf diphtheria toxoid; and 67 Lf tetanus toxoid. As an alternative, the concentrations of toxoids may be fractions of these values, provided that the relative proportions (the ratio) stay the same *e.g*. as obtainable by simple dilution.

The first bulk may include further compounds in addition to the toxoids, adjuvant and optional preservative. In particular, it may comprise sodium chloride and/or residual formaldehyde. A sodium chloride concentration of between 5mg and 6mg per 100 Lf of diphtheria toxoid is preferred *e.g.* between 8 and 9 mg/ml (*e.g.* about 8.5 mg/ml).

The first bulk is preferably free from polysorbate 80.

### The second aqueous bulk

The second bulk used in the processes of the invention comprises tetanus toxoid, but is free from diphtheria toxoid.

The tetanus toxoid in the second bulk may be adsorbed onto an aluminum hydroxide adjuvant. Preferably, however, the tetanus toxoid is free from adjuvants, and in particular is free from aluminium salt(s). If the tetanus toxoid in the second bulk is adsorbed to an aluminium hydroxide adjuvant then a process of the invention may involve an initial step of: preparing the second bulk by combining an aluminium-free tetanus toxoid preparation with an aluminium hydroxide adjuvant. Prior to being used in a process of the invention, the combined adjuvant and toxoid may be left for a period to allow adsorption.

The second bulk may include a preservative, such as 1-hydroxy-2-phenoxyethane. Preferred bulks, however, are free from preservatives, and in particular are free from 1-hydroxy-2-phenoxyethane. The second bulk is free from mercurial preservatives (*e.g.* thimerosal).

The second bulk is preferably free from polysorbate 80.

### Diphtheria toxoids

Diphtheria is caused by *Corynebacterium diphtheriae,* a Gram-positive non-sporing aerobic bacterium. This organism expresses a prophage-encoded ADP-ribosylating exotoxin ('diphtheria toxin'), which can be treated *(e.g.* using formaldehyde) to give a toxoid that is no longer toxic but that remains antigenic and is able to stimulate the production of specific anti-toxin antibodies after injection. Diphtheria toxoids are disclosed in more detail in chapter 13 of reference 2. Preferred diphtheria toxoids are those prepared by formaldehyde treatment. The diphtheria toxoid can be obtained by growing *C.diphtheriae* in growth medium (*e.g.* Fenton medium, or Linggoud & Fenton medium), which may be supplemented with bovine extract, followed by formaldehyde treatment, ultrafiltration and precipitation. The toxoided material may then be treated by a process comprising sterile filtration and/or dialysis.

Quantities of diphtheria toxoid can be expressed in international units (IU). For example, the NIBSC [3] supplies the 'Diphtheria Toxoid Adsorbed Third International Standard 1999' [4,5], which contains 160 IU per ampoule. As an alternative to the IU system, the 'Lf' unit ("flocculating units", the "limes flocculating dose", or the "limit of flocculation") is defined as the amount of toxoid which, when mixed with one International Unit of antitoxin, produces an optimally flocculating mixture [6]. For example, the NIBSC supplies 'Diphtheria Toxoid, Plain' [7], which contains 300 Lf per ampoule, 'The 1 st International Reference Reagent For Diphtheria Toxoid For Flocculation Test' [8] which contains 900 Lf per ampoule. The conversion between IU and Lf systems depends on the particular toxoid preparation.

Where bovine materials are used in the culture of *C.diphtheriae,* they should be obtained from sources that are free from bovine spongiform encephalopathy (BSE) or from other transmissible spongiform encephalopathies (TSEs).

### Tetanus toxoids

Tetanus is caused by *Clostridium tetani,* a Gram-positive, spore-forming bacillus. This organism expresses an endopeptidase ('tetanus toxin'), which can be treated to give a toxoid that is no longer toxic but that remains antigenic and is able to stimulate the production of specific anti-toxin antibodies after injection. Tetanus toxoids are disclosed in more detail in chapter 27 of reference 2. Preferred tetanus toxoids are those prepared by formaldehyde treatment. The tetanus toxoid can be obtained by growing *C.tetani* in growth medium *(e.g.* a Latham medium derived from bovine casein), followed by formaldehyde treatment, ultrafiltration and precipitation. The material may then be treated by a process comprising sterile filtration and/or dialysis.

Quantities of tetanus toxoid can be expressed in international units (IU). For example, the NIBSC [9] supplies the 'Tetanus Toxoid Adsorbed Third International Standard 2000' [10,11], which contains 469 IU per ampoule. As an alternative to the IU system, the 'Lf' unit is defined as the amount of toxoid which, when mixed with one International Unit of antitoxin, produces an optimally flocculating mixture [12]. For example, the NIBSC supplies 'The 1st International Reference Reagent for Tetanus Toxoid For Flocculation Test' [13] which contains 1000 LF per ampoule. The conversion between IU and Lf systems depends on the particular toxoid preparation.

Where bovine materials are used in the culture of *C.tetani*, they should be obtained from sources that are free from bovine spongiform encephalopathy (BSE) or from other transmissible spongiform encephalopathies (TSEs).

### Acellular pertussis antigens

*Bordetella pertussis* is a Gram-negative non-sporing aerobic bacterium that causes whooping cough. As described in more detail in chapter 21 of reference 2, vaccines against *B.pertussis* have been available for many years, and fall into two categories: cellular and acellular. Cellular vaccines comprise whole *B.pertussis* cells which have been killed and deactivated *(e.g.* by treatment with formalin and/or heat), whereas acellular vaccines comprise specific purified *B.pertussis* antigens, either purified from the native bacterium or purified after expression in a recombinant host.

Processes of the invention may involve the use of acellular pertussis antigens in order to provide vaccines comprising diphtheria toxoid, tetanus toxoid and acellular pertussis antigens.

The invention will typically use more than one acellular pertussis (aP) antigen in a single vaccine *e.g.* two or three of the following well-known and well-characterized *B.pertussis* antigens: (1) detoxified pertussis toxin (pertussis toxoid, or 'PT'); (2) filamentous hemagglutinin ('FHA'); (3) pertactin (also known as the '69 kiloDalton outer membrane protein'). It is most preferred that all three of these antigens should be used. These three antigens are preferably prepared by isolation from *B.pertussis* culture grown in modified Stainer-Scholte liquid medium. PT and FHA can be isolated from the fermentation broth (*e.g.* by adsorption on hydroxyapatite gel), whereas pertactin can be extracted from the cells by heat treatment and flocculation (*e.g.* using barium chloride). The antigens can be purified in successive chromatographic and/or precipitation steps. PT and FHA can be purified by hydrophobic chromatography, affinity chromatography and size exclusion chromatography. Pertactin can be purified by ion exchange chromatography, hydrophobic chromatography and size exclusion chromatography.

FHA and pertactin may be treated with formaldehyde prior to use according to the invention. PT is preferably detoxified by treatment with formaldehyde and/or glutaraldehyde. As an alternative to this chemical detoxification procedure the PT may be a mutant PT in which enzymatic activity has been reduced by mutagenesis [14], but detoxification by chemical treatment is preferred.

Further acellular pertussis antigens that can be used include fimbriae (*e.g.* agglutinogens 2 and 3).

The aP antigen(s) may be used in an unadsorbed state, but they are preferably adsorbed onto one or more aluminum salt adjuvant(s) before being used. The aP antigens are preferably adsorbed onto an aluminum hydroxide adjuvant.

The aP antigens can be added to the first and/or second bulk(s), or they may be added as a separate bulk. Where more than one aP antigen is used, each aP antigen may be added as its own bulk, or aP antigens may be combined into the same bulk prior to use.

Preferably, aP components are substantially free from 1-hydroxy-2-phenoxyethane.

Preferably, aP components are substantially free from mercurial preservatives (*e.g.* thimerosal).

The aP component(s) may include polysorbate 80. The level of polysorbate 80 in the pertussis component(s) is such that, after any dilution of the component to ensure that the pertussis antigens are at the concentration desired in the final vaccine product, the polysorbate 80 concentration is less than 200µg/ml, and is preferably less than 100 µg/ml. A typical polysorbate concentration in the final vaccine product is between 70 and 90 µg/ml *e.g*. about 80µg/ml.

A preferred upper limit on the amount of polysorbate 80 in an aP component prior to its use in the process of the invention is <40 µg per 10 µg of FHA.

Quantities of acellular pertussis antigens are typically expressed in micrograms. For a combination of PT, FHA and pertactin, the amounts of each antigen are preferably selected to give a final PT:FHA:pertactin weight ratio in the final vaccine of 8 : 8 : 2.5.

### Inactivated poliovirus antigens

Poliomyelitis can be caused by one of three types of poliovirus. The three types are similar and cause identical symptoms, but they are antigenically very different and infection by one type does not protect against infection by others. As explained in chapter 24 of reference 2, it is therefore preferred to use three poliovirus antigens in the process of the invention - poliovirus Type 1 (*e.g.* Mahoney strain), poliovirus Type 2 (*e.g.* MEF-1 strain), and poliovirus Type 3 (*e.g.* Saukett strain).

Processes of the invention may involve the use of inactivated poliovirus (IPV) antigens in order to provide vaccines comprising diphtheria toxoid, tetanus toxoid and inactivated poliovirus antigens.

Polioviruses may be grown in cell culture. A preferred culture uses a Vero cell line, which is a continuous cell line derived from monkey kidney. Vero cells can conveniently be cultured microcarriers. Culture of the Vero cells before and during viral infection may involve the use of bovine-derived material, such as calf serum, and of lactalbumin hydrolysate (*e.g.* obtained by enzymatic degradation of lactalbumin). Such bovine-derived material should be obtained from sources which are free from BSE or other TSEs. After growth, virions may be purified using techniques such as ultrafiltration, diafiltration, and chromatography. Prior to administration to patients, polioviruses must be inactivated, and this can be achieved by treatment with formaldehyde before the viruses are used in the process of the invention.

The three polioviruses are preferably grown, purified and inactivated individually, and are then combined to give a bulk mixture for use in the process of the invention. The combined polioviruses are preferably not adsorbed to any adjuvant before being used in the process of the invention, but after the addition they may become adsorbed onto aluminum adjuvant(s) already present in the vaccine composition.

The IPV antigens can be added to the first and/or second bulk(s), or they may be added as a separate bulk.

Preferably, IPV antigens are substantially free from 1-hydroxy-2-phenoxyethane.

Preferably, IPV antigens are substantially free from mercurial preservatives (*e.g.* thimerosal).

Preferably, IPV antigens are substantially free from polysorbate 80 *e.g.* they contain less than 0.1 µg/ml of polysorbate 80, and preferably contain no detectable polysorbate 80. Polysorbate 80 may, however, have been used during production of an IPV component.

IPV antigens may include trace amounts of neomycin and/or polymyxin.

Quantities of IPV antigens are typically expressed in the 'DU' unit (the "D-antigen unit" [15]). The amounts of antigen for each separate strain are preferably selected to give a type 1:2:3 DU ratio in the final vaccine of 5 : 1 : 4.

### Further optional antigens

The invention is preferably used to prepare 3-valent diphtheria-tetanus-pertussis vaccines or to prepare 4-valent diphtheria-tetanus-pertussis-poliovirus vaccines. In addition to diphtheria, tetanus, pertussis and IPV antigens, however, vaccines of the invention may include one or more further antigens *e.g.* from hepatitis B virus, from *Haemophilus influenzae* B, from *Streptococcus pneumoniae,* from *Neisseria meningitidis,* hepatitis A virus, measles virus, mumps virus, rubella virus, rotavirus, influenza virus, *etc.*

### Adjuvants

As described above, antigens may be adsorbed to aluminum adjuvants prior to being used in the process of the invention (they are 'pre-adsorbed').

Aluminum adjuvants currently in use are typically referred to either as "aluminum hydroxide" or as "aluminum phosphate" adjuvants. These are names of convenience, however, as neither is a precise description of the actual chemical compound which is present (*e.g.* see chapter 9 of reference 16). The invention can use any of the "hydroxide" or "phosphate" adjuvants that are in general use as adjuvants.

The adjuvants known as "aluminum hydroxide" are typically aluminum oxyhydroxide salts, which are usually at least partially crystalline. Aluminum oxyhydroxide, which can be represented by the formula AlO(OH), can be distinguished from other aluminum compounds, such as aluminum hydroxide Al(OH)₃, by infrared -(IR) spectroscopy, in particular by the presence of an adsorption band at 1070cm⁻¹ and a strong shoulder at 3090-3100cm⁻¹ [chapter 9 of ref. 16]. The degree of crystallinity of an aluminium hydroxide adjuvant is reflected by the width of the diffraction band at half height (WHH), with poorly-crystalline particles showing greater line broadening due to smaller crystallite sizes. The surface area increases as WHH increases, and adjuvants with higher WHH values have been seen to have greater capacity for antigen adsorption. A fibrous morphology (*e.g.* as seen in transmission electron micrographs) is typical for aluminium hydroxide adjuvants. The pI of aluminium hydroxide adjuvants is typically about 11 *i.e.* the adjuvant itself has a positive surface charge at physiological pH. Adsorptive capacities of between 1.8-2.6 mg protein per mg Al⁺⁺⁺ at pH 7.4 have been reported for aluminium hydroxide adjuvants.

The adjuvants known as "aluminum phosphate" are typically aluminum hydroxyphosphates, often also containing a small amount of sulfate. They may be obtained by precipitation, and the reaction conditions and concentrations during precipitation influence the degree of substitution of phosphate for hydroxyl in the salt. Hydroxyphosphates generally have a PO₄/Al molar ratio between 0.3 and 0.99. Hydroxyphosphates can be distinguished from strict AlPO₄ by the presence of hydroxyl groups. For example, an IR spectrum band at 3164cm⁻¹ (*e.g*. when heated to 200°C) indicates the presence of structural hydroxyls [chapter 9 of ref. 16].

The PO₄/Al³⁺ molar ratio of an aluminium phosphate adjuvant will generally be between 0.3 and 1.2, preferably between 0.8 and 1.2, and more preferably 0.95±0.1. The aluminium phosphate will generally be amorphous, particularly for hydroxyphosphate salts. A typical adjuvant is amorphous aluminium hydroxyphosphate with PO₄/Al molar ratio between 0.84 and 0.92, included at 0.6mg Al³⁺/ml. The aluminium phosphate will generally be particulate (*e.g.* plate-like morphology as seen in transmission electron micrographs). Typical diameters of the particles are in the range 0.5-20µm (*e.g*. about 5-10µm) after any antigen adsorption. Adsorptive capacities of between 0.7-1.5 mg protein per mg Al⁺⁺⁺ at pH 7.4 have been reported for aluminium phosphate adjuvants.

The point of zero charge (PZC) of aluminium phosphate is inversely related to the degree of substitution of phosphate for hydroxyl, and this degree of substitution can vary depending on reaction conditions and concentration of reactants used for preparing the salt by precipitation. PZC is also altered by changing the concentration of free phosphate ions in solution (more phosphate = more acidic PZC) or by adding a buffer such as a histidine buffer (makes PZC more basic). Aluminium phosphates used according to the invention will generally have a PZC of between 4.0 and 7.0, more preferably between 5.0 and 6.5 e.g. about 5.7.

Vaccines of the invention may have an aluminium hydroxide adjuvant, or they may have a combined aluminium hydroxide/aluminium phosphate adjuvant. Where both a hydroxide and a phosphate salt are present, the ratio of Al³⁺ content provided by each is preferably 3 : 2 (*e.g.* 0.6mg/ml Al³⁺ from aluminium hydroxide, and 0.4mg/mlAl³⁺ from aluminium phosphate).

The Al³⁺ content of adolescent booster vaccines may be important for optimum immunity [17]. The total aluminium content of a vaccine produced by the invention is (measured by Al³⁺) preferably more than 0.25mg/ml, but is preferably less than 1.5mg/ml *e.g.* it is about 0.6mg/ml.

To give a desired final Al³⁺ content, it is possible to use an appropriate Al³⁺ content of bulks prior to their being combined and/or to add extra aluminium salt(s) as a separate bulk *e.g.* to combine the first bulk, the second bulk and a third bulk, wherein the third bulk contains aluminium adjuvant(s).

### Vaccines produced by processes of the invention

In typical use, processes of the invention will be used to provide bulk combination vaccine which is suitable for packaging, and then for distribution and administration. Concentrations mentioned above are typically concentrations in final packaged vaccine, and so concentrations in bulk vaccine may be higher (*e.g.* to be reduced to final concentrations by dilution).

Processes of the invention may therefore comprise the further step of packaging the vaccine into containers for use. Containers may include a single dose of vaccine, or they may include more than one dose (a 'multidose' container) *e.g.* 10 doses. When using a multidose vial, each dose should be withdrawn with a sterile needle and syringe under strict aseptic conditions, taking care to avoid contaminating the vial contents. Single dose containers are preferred. Multiple single-dose containers can be packaged together *e.g.* 10 single dose vials.

Suitable containers include vials and disposable syringes. These are preferably sterile. Packaging into syringes, such that the vaccine is ready for administration by a final user, is particularly preferred. Syringes may be distributed with an attached needle, but it is also possible to distribute them needle-free.

Syringes may be provided with peel-off labels on which the lot number and expiration date of the contents may be printed (*e.g.* as a barcode), to facilitate record keeping. A syringe may include a coloured (*e.g.* green) band to facilitate identification of its contents. The plunger in the syringe preferably has a stopper to prevent the plunger from being accidentally removed during aspiration. The syringes may have a latex rubber cap and/or plunger. A syringe will generally have a tip cap to seal the tip prior to attachment of a needle, and the tip cap is preferably made of butyl rubber. If the syringe and needle are packaged separately then the needle is preferable fitted with a butyl rubber shield. Grey butyl rubber is preferred. The syringe may have a tip incorporating a locking mechanism for securing a needle *e.g.* a Luer-Lok™. Suitable syringes are those marketed under the trade name "Tip-Lok™ or Hypak™ PRTC.

Where the vaccine is packaged into vials, these may be made of a plastic material or, preferably, from a glass. The vial is preferably sterilized before vaccine is added to it. To avoid problems with latex-sensitive patients, vials are preferably sealed with a latex-free stopper.

After vaccine is placed into a container, the container may then be enclosed within a box for distribution *e.g.* inside a cardboard box. A box will be labeled with details of the vaccine *e.g.* its trade name, a list of the antigens in the vaccine (*e.g.* 'Tetanus Toxoid, Reduced Diphtheria Toxoid and Acellular Pertussis Vaccine, Adsorbed', and/or 'Tdap'), the presentation container (*e.g.* 'Disposable Prefilled Tip-Lok Syringes' or '10 x 0.5 ml Single-Dose Vials'), its dose (*e.g.* 'each containing a single 0.5ml dose'), warnings (*e.g.* 'For Adolescent Use Only'), an expiration date, the manufacturer's name, *etc.* Each box might contain more than one packaged vaccine *e.g.* five or ten packaged vaccines, such as five syringes or ten vials. For visual effect, the background colour of one corner of a box may be a different colour from the rest of the box *e.g.* a purple corner on a green box.

The vaccine may be packaged together (*e.g.* in the same box) with a leaflet including details of the vaccine e.g. instructions for administration, details of the antigens within the vaccine, etc. The instructions may also contain warnings *e.g.* to keep a solution of adrenaline readily available in case of anaphylactic reaction following vaccination, *etc*.

Processes of the invention are particularly useful for preparing vaccines with the following content per milliliter:

| **Antigen** | **Concentration per ml** * |
|---|---|
| Diphtheria toxoid | 5 Lf |
| Tetanus toxoid | 10 Lf |
| Inactivated pertussis toxin | 16 µg |
| Filamentous hemagglutinin | 16 µg |
| Pertactin | 5 µg |
| Type 1 poliovirus** | 80 DU |
| Type 2 poliovirus** | 16 DU |
| Type 3 poliovirus** | 64 DU |

| | |
|---|---|
| * These doses are given ±10% ** In a 4-valent D-T-aP-IPV vaccine | |

The concentration of individual antigens in the bulk vaccine, the dilutions required before addition of antigens to the bulk vaccine, and any dilution required from bulk to final vaccine can be calculated accordingly, and thus the process of the invention may performed to produce a final combination vaccine which contains antigens at these concentrations per dose.

The final vaccine is preferably sterile.

The final vaccine is preferably non-pyrogenic *e.g.* it contains <1 EU (endotoxin unit; 1 EU is equal to 0.2 ng FDA reference standard Endotoxin EC-2 'RSE') per dose, and preferably <0.1 EU per dose.

The final vaccine is preferably gluten free.

The pH of the final vaccine is preferably between 6 and 8 *e.g.* between 6.5 and 7.5. A process of the invention may therefore include a step of adjusting the pH of the bulk vaccine prior to packaging.

The packaged vaccine is preferably a turbid white suspension.

The packaged vaccine is preferably stored at between 2°C and 8°C. It should not be frozen.

To control tonicity of the final vaccine product, it is preferred to include a physiological salt, such as a sodium salt. Sodium chloride (NaCl) is preferred, such as isotonic sodium chloride. A final NaCl concentration of 9mg/ml is usually suitable.

It is preferred not to use preservatives, and particularly mercurial preservatives (*e.g.* thimerosal) during the process of the invention.

### Adininistration of the vaccines

The combination vaccines produced by the processes of the invention are preferably administered to patients by injection. Intramuscular injection is preferred (*e.g.* into the deltoid muscle of the upper arm). Before injection, the skin at the injection site should ideally be cleaned and prepared with a suitable germicide A typical injection will have a volume of 0.5ml, and so a process of the invention may comprise a step of extracting and packaging a 0.5ml sample of a bulk combination vaccine into a container. A typical needle size for administration of the vaccine BOOSTRIX is a 22-25 gauge needle, between 1 and 1.25 inches in length (2.5 to 3.2 cm).

Where a vaccine contains an aluminium salt adjuvant, it should be shaken vigorously to obtain a homogeneous, turbid, white suspension before administration.

The vaccine is ideally administered as a booster vaccine to a patient who has previously been vaccinated against both diphtheria and tetanus, and preferably also against pertussis. These patients can be distinguished from the general population by having an immunological memory response against the previous vaccine. Preferred patients received their most recent diphtheria and/or tetanus vaccines at least five years before receiving the vaccine of the invention. Preferred patients receiving the vaccines are thus aged between 4 and 65 years of age *e.g.* 11-64 years, 10-18 years, *etc.*

Vaccines produced by the invention may be administered to patients at substantially the same time as (*e.g.* during the same medical consultation or visit to a healthcare professional or vaccination centre) other vaccines *e.g.* at substantially the same time as a meningococcal conjugate vaccine (such as a tetravalent serogroup A-C-W135-Y vaccine) and/or a multivalent pneumococcal conjugate vaccine, *etc.* Such further vaccines should be given with separate syringes and at different injection sites from the vaccines of the invention. The vaccines may similarly be administered at substantially the same time as tetanus immunoglobulin.

For any particular antigen, vaccination guidelines *(e.g.* WHO guidelines) may recommend or prescribe a quantity that should be present in a final vaccine. That information (*e.g.* 10 µg per dose) can be combined with the volume of a vaccine dose (*e.g.* a 0.5 ml dose) to calculate the concentration required in the bulk vaccine for that antigen.

Processes of the invention are particularly useful for preparing vaccines with the following content per 0.5ml dose:

| **Antigen** | **Concentration per dose*** |
|---|---|
| Diphtheria toxoid | 2.5 Lf |
| Tetanus toxoid | 5 Lf |
| Inactivated pertussis toxin | 8 µg |
| Filamentous hemagglutinin | 8 µg |
| Pertactin | 2.5 µg |
| Type 1 poliovirus** | 40 DU |
| Type 2 poliovirus** | 8 DU |
| Type 3 poliovirus** | 32 DU |

| | |
|---|---|
| * These doses are given ±10% ** In a 4-valent D-T-aP-IPV vaccine | |

### General

The term "comprising" encompasses "including" as well as "consisting" *e.g.* a composition "comprising" X may consist exclusively of X or may include something additional *e.g*. X + Y.

The word "substantially" does not exclude "completely" *e.g.* a composition which is "substantially free" from Y may be completely free from Y. Where necessary, the word "substantially" may be omitted from the definition of the invention.

The term "about" in relation to a numerical value *x* means, for example, *x*±10%.

Where a glass container is used, a borosilicate glass is preferred to a soda lime glass.

Where a component is described as being "adsorbed" to an adjuvant, it is preferred that at least 50% (by weight) of that antigen is adsorbed *e.g*. 50%, 60%, 70%, 80%, 90%, 95%, 98% or more. If a component is totally adsorbed then none should detectable in the supernatant of a composition after centrifugation.

### BRIEF DESCRIPTION OF THE DRAWINGS

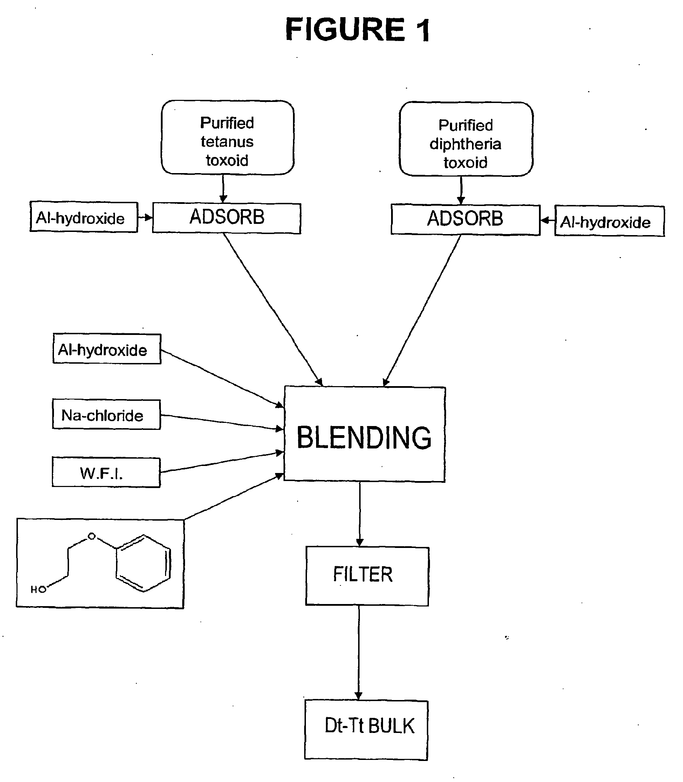

Figure 1 illustrates the preparation of a mixed bulk of adsorbed diphtheria and tetanus toxoids.

### EXAMPLE

### Example 1/D-T-aP vaccine for use as adolescent booster

A first bulk antigen component was prepared as shown in Figure 1. It has the following composition:

| **Component** | **Concentration per ml*** |
|---|---|
| Diphtheria toxoid | 167 Lf |
| Tetanus toxoid | 67 Lf |
| Aluminium hydroxide adjuvant | 6.91 mg |
| 1-hydroxy-2-phenoxyethane | 5 mg |
| Sodium chloride | 8.5 mg |
| pH | Between 6.0 and 6.3 |

| | |
|---|---|
| * These doses are given ±10% | |

The diphtheria and tetanus toxoids were both fully adsorbed to the adjuvant, with none detectable in the supernatant after centrifugation of a sample of the bulk.

This bulk can be used to prepare pediatric D-T-aP vaccines *e.g.* containing 25Lf of diphtheria toxoid and 10Lf of tetanus toxoid per dose. For an adolescent booster dose requiring more tetanus toxoid than diphtheria toxoid (as measured in Lf units), however, this bulk cannot be used. If the bulk is diluted to a desired final diphtheria toxoid dose of 2.5 Lf then the diluted tetanus toxoid dose will be only 1Lf, which is 5 times lower than desired. The diluted Al³⁺ dose (<0.1mg) will also be lower than the minimum effective dose reported in reference 17. The diphtheria : tetanus ratio and the Al³⁺ dose thus have to be modified.

To permit adjustment of the diphtheria: tetanus ratio, a second bulk was prepared including concentrated tetanus toxoid (between 3000 Lf/ml and 3000 Lf/ml; sodium chloride between 8mg/ml and 9mg/ml; pH between 7.0 and 7.8). The second bulk has no other antigens, no adjuvant and no preservative.

These two antigen bulks are combined and diluted, together with bulks including additional aluminium adjuvants (hydroxide and phosphate), acellular pertussis antigens adsorbed to aluminium hydroxide adjuvant, and sodium chloride, to give a bulk combination vaccine with the final desired concentrations and ratios of components. This procedure can be used to prepare a bulk with the following composition per ml:

| **Component** | **Amount per ml *** |
|---|---|
| Diphtheria toxoid | 5 Lf |
| Tetanus toxoid | 10 Lf |
| Inactivated pertussis toxin | 16 µg |
| Filamentous hemagglutinin | 16 µg |
| Pertactin | 5 µg |
| Al³⁺ | 1 mg |
| | 0.6 mg from aluminium hydroxide |
| | 0.4 mg from aluminium phosphate |

This bulk is packaged in 0.5ml doses into Tip-Lok™ syringes. This vaccine can be used as a booster in adolescents who previously received D-T-P vaccines.

Thus the first bulk can be used as the base of both pediatric and adolescent D-T-P vaccines.

### Example 2/Preservative-free reduced-aluminium D-T-aP vaccine

To eliminate the preservative in the vaccine prepared in example 1, a different first bulk is used. No 1-hydroxy-2-phenoxyethane is added during the blending step.

To reduce the aluminium content, no aluminium phosphate adjuvant is added.

After packaging, each preservative-free 0.5ml dose has following composition:

| **Component** | **Amount per dose*** |
|---|---|
| Diphtheria toxoid | 2.5 Lf |
| Tetanus toxoid | 5 Lf |
| Inactivated pertussis toxin | 8 µg |
| Filamentous hemagglutinin | 8 µg |
| Pertactin | 2.5 µg |
| Sodium chloride | 4.5 mg |
| Al³⁺ | 0.3 mg |

| | |
|---|---|
| * These doses are given ±10% | |

### Example 3/ D-T-aP-IPV vaccine

To make a 4-valent D-T-aP-IPV vaccine, bulk mixed IPV is added to the process of example 1, to give a final vaccine with the following content per 0.5ml dose:

| **Component** | **Amount per dose*** |
|---|---|
| Diphtheria toxoid | 2.5 Lf |
| Tetanus toxoid | 5 Lf |
| Inactivated pertussis toxin | 8 µg |
| Filamentous hemagglutinin | 8 µg |
| Pertactin | 2.5 µg |
| Poliovirus type 1 (Mahoney) | 40 DU |
| Poliovirus type 2 (MEF1) | 8 DU |
| Poliovirus type 3 (Saukett) | 32 DU |
| Al³⁺ | 0.5 mg |
| | 0.3 mg from aluminium hydroxide |
| | 0.2 mg from aluminium phosphate |

| | |
|---|---|
| * These doses are given ±10% | |

It will be understood that the invention has been described by way of example only and modifications may be made whilst remaining within the scope and spirit of the invention.

### REFERENCES (the contents of which are hereby incorporated by reference)

[1] Broder et al. (2006) MMWR Recomm Rep 55(RR-3):1-34.
[2] Vaccines. (eds. Plotkin & Orenstein). 4th edition, 2004, ISBN: 0-7216-9688-0.
[3] *National Institute for Biological Standards and Control;* Potters Bar, UK. *www.nibsc.ac.uk*
[4] Sesardic et al. (2001) Biologicals 29:107-22.
[5] NIBSC code: 98/560.
[6] Module 1 of WHO's *The immunological basis for immunization series* (Galazka).
[7] NIBSC code: 69/017.
[8] NIBSC code: DIFT.
[9] *National Institute for Biological Standards and Control;* Potters Bar, UK. *www.nibsc.ac.uk*
[10] Sesardic et al. (2002) Biologicals 30:49-68.
[11] NIBSC code: 98/552.
[12] Module 1 of WHO's *The immunological basis for immunization series* (Galazka).
[13] NIBSC code: TEFT.
[14] Rappuoli et al. (1991) TIBTECH 9:232-238.
[15] Module 6 of WHO's *The immunological basis for inmmunization series* (Robertson)
[16] Vaccine Design: The Subunit and Adjuvant Approach (eds. Powell & Newman) Plenum Press 1995 (ISBN 0-306-44867-X).
[17] Theeten e al. (2005) Vaccine 23:1515-21.

## Claims

1. A process for manufacturing a vaccine, wherein the vaccine comprises diphtheria toxoid and tetanus toxoid, and wherein the process comprises steps of combining (i) a first bulk comprising diphtheria toxoid and tetanus toxoid at a diphtheria : tetanus ratio between 2:1 and 3:1 (measured in Lf units) with (ii) a second bulk comprising tetanus toxoid but no diphtheria toxoid.

2. The process of claim 1, wherein the vaccine also comprises one or more acellular pertussis antigens *e.g.* including inactivated pertussis toxin, filamentous hemagglutinin and pertactin.

3. The process of claim 2, wherein inactivated pertussis toxin, filamentous hemagglutinin and pertactin are present at a ratio of 8 : 8 : 3 (measured by weight).

4. The process of any preceding claim, wherein the vaccine also comprises inactivated poliovirus (IPV) antigens *e.g.* IPV antigens from each of a poliovirus Type 1 strain, a poliovirus Type 2 strain and a poliovirus Type 3 strain.

5. The process of claim 4, wherein the IPV antigens are present at a Type 1:2:3 ratio of 5 : 1 : 4 (measured by DU).

6. The process of any preceding claim, wherein the vaccine comprises one or more aluminium salt adjuvants *e.g.* wherein the vaccine comprises an aluminium hydroxide adjuvant and, optionally, further comprises an aluminium phosphate adjuvant.

7. The process of claim 6, wherein the vaccine comprises no aluminium phosphate adjuvant.

8. The process of any preceding claim, wherein the diphtheria and tetanus toxoids in the first bulk are adsorbed onto an aluminum hydroxide adjuvant.

9. The process of any preceding claim, wherein the first bulk is free from mercurial preservatives.

10. The process of any preceding claim, wherein the first bulk includes a preservative *e.g.* 1-hydroxy-2-phenoxyethane.

11. The process of claim 10, wherein the first bulk includes about 5 g/l 1-hydroxy-2-phenoxyethane.

12. The process of any one of claims 1 to 9, wherein the first bulk is preservative-free.

13. The process of any preceding claim, wherein the diphtheria toxoid and tetanus toxoid in the first bulk are present at a diphtheria : tetanus ratio of about 2.5 : 1 (measured in Lf units).

14. The process of any preceding claim, wherein the first bulk includes between 5mg and 6mg of sodium chloride per 100 Lf of diphtheria toxoid and/or is free from polysorbate 80.

15. The process of any preceding claim, wherein the second bulk is free from aluminium salts and/or is free from mercurial preservatives and/or is preservative-free and/or is free from polysorbate 80.

16. The process of any preceding claim, wherein the vaccine has an Al³⁺ content of between 0.25mg/ml and 1.5mg/ml *e.g.* an Al³⁺ content of about 0.6mg/ml.

17. The process of any preceding claim, further comprising a step of packaging the vaccine into containers *e.g.* wherein the container is a vial or wherein the container is a syringe.

18. The process of any one of claims 2 to 17, wherein the vaccine has (i) the following content per milliliter: 5 Lf diphtheria toxoid; 10 Lf tetanus toxoid; 16 µg inactivated pertussis toxin; 16 µg filamentous hemagglutinin; 5 µg pertactin' or (ii) the following content per milliliter: 5 Lf diphtheria toxoid; 10 Lf tetanus toxoid; 16 µg inactivated pertussis toxin; 16 µg filamentous hemagglutinin; 5 µg pertactin; 80 DU Mahoney strain poliovirus; 16 DU MEF1 strain poliovirus; and 64 DU Saukett strain poliovirus.

19. The process of any preceding claim, wherein the vaccine is preservative-free.

## Patentansprüche

1. Verfahren zur Herstellung eines Impfstoffs, wobei der Impfstoff ein Diphtherie-Toxoid und ein Tetanus-Toxoid umfasst, und wobei das Verfahren Schritte umfasst, bei denen man (i) eine erste Menge (bulk), die Diphtherie-Toxoid und Tetanus-Toxoid in einem Diphtherie:Tetanus-Verhältnis von zwischen 2:1 und 3:1 (gemessen in Lf-Einheiten) umfasst, mit (ii) einer zweiten Menge (bulk), welcher Tetanus-Toxoid aber kein Diphtherie-Toxoid umfasst, kombiniert.

2. Verfahren nach Anspruch 1, wobei der Impfstoff ferner ein oder mehrere azelluläre Pertussis-Antigene umfasst, einschließlich z.B. inaktiviertes Pertussis-Toxin, filamentöses Hämagglutinin und Pertactin.

3. Verfahren nach Anspruch 2, wobei inaktiviertes Pertussis-Toxin, filamentöses Hämagglutinin und Pertactin in einem Verhältnis von 8:8:3 vorliegen (gemessen nach Gewicht).

4. Verfahren nach einem der vorhergehenden Ansprüche, wobei der Impfstoff ferner inaktivierte Antigene von Poliovirus (IPV) umfasst, z.B. IPV-Antigene von jeweils einem Poliovirus Typ 1-Stamm, einem Poliovirus Typ 2-Stamm und einem Poliovirus Typ 3-Stamm.

5. Verfahren nach Anspruch 4, wobei die IPV-Antigene in einem Typ-1:2:3-Verhältnis von 5:1:4 vorliegen (gemessen in DU).

6. Verfahren nach einem der vorhergehenden Ansprüche, wobei der Impfstoff ein oder mehrere Aluminiumsalz-Adjuvantien umfasst, z.B. wobei der Impfstoff ein Aluminium-Hydroxid-Adjuvans umfasst und darüber hinaus wahlweise ein Aluminium-Phosphat-Adjuvans.

7. Verfahren nach Anspruch 6, wobei der Impfstoff kein Aluminium-Phosphat-Adjuvans umfasst.

8. Verfahren nach einem der vorhergehenden Ansprüche, wobei die Diphtherie- und Tetanus-Toxoide in der ersten Menge (bulk) an ein Aluminium-Hydroxid-Adjuvans adsorbiert sind.

9. Verfahren nach einem der vorhergehenden Ansprüche, wobei die erste Menge (bulk) frei von Quecksilber-Konservierungsmitteln ist.

10. Verfahren nach einem der vorhergehenden Ansprüche, wobei die erste Menge (bulk) ein Konservierungsmittel enthält, wie z.B. 1-Hydroxy-2-Phenoxyethan.

11. Verfahren nach Anspruch 10, wobei die erste Menge (bulk) etwa 5 g/L 1-Hydroxy-2-Phenoxyethan enthält.

12. Verfahren nach einem der Ansprüche 1 bis 9, wobei die erste Menge (bulk) frei von Konservierungsmitteln ist.

13. Verfahren nach einem der vorhergehenden Ansprüche, wobei das Diphtherie-Toxoid und das Tetanus-Toxoid in der ersten Menge (bulk) in einem Diphtherie-Tetanus-Verhältnis von etwa 2,5:1 vorliegen (gemessen in Lf-Einheiten).

14. Verfahren nach einem der vorhergehenden Ansprüche, wobei die erste Menge (bulk) zwischen 5 mg und 6 mg Natriumchlorid pro 100 Lf Diphtherie-Toxoid enthält und/oder frei von Polysorbat 80 ist.

15. Verfahren nach einem der vorhergehenden Ansprüche, wobei die zweite Menge (bulk) frei von Aluminiumsalzen und/oder frei von Quecksilber-Konservierungsmitteln und/oder frei von Konservierungsmitteln und/oder frei von Polysorbat 80 ist.

16. Verfahren nach einem der vorhergehenden Ansprüche, wobei der Impfstoff einen Gehalt an Al³⁺ von zwischen 0,25 mg/ml und 1,5 mg/ml aufweist, z.B. einen Gehalt an Al³⁺ von etwa 0,6 mg/ml.

17. Verfahren nach einem der vorhergehenden Ansprüche, bei dem man ferner den Impfstoff in Behälter verpackt, wobei z.B. der Behälter eine Ampulle/Glasfläschchen ist oder wobei der Behälter eine Spritze ist.

18. Verfahren nach einem der Ansprüche 2 bis 17, wobei der Impfstoff (i) den folgenden Gehalt pro Milliliter aufweist: 5 Lf Diphtherie-Toxoid; 10 Lf Tetanus-Toxoid; 16 µg inaktiviertes Pertussis-Toxin; 16 µg filamentöses Hämagglutinin; 5 µg Pertactin, oder (ii) den folgenden Gehalt pro Milliliter: 5 Lf Diphtherie-Toxoid; 10 Lf Tetanus-Toxoid; 16 µg inaktiviertes Pertussis-Toxin; 16 µg filamentöses Hämagglutinin; 5 µg Pertactin, 80 DU Mahoney-Stamm-Poliovirus, 16 DU MEF1-Stamm-Poliovirus und 64 DU Saukett-Stamm-Poliovirus.

19. Verfahren nach einem der vorhergehenden Ansprüche, wobei der Impfstoff frei von Konservierungsmitteln ist.

## Revendications

1. Processus pour fabriquer un vaccin, dans lequel le vaccin comprend l'anatoxine diphtérique et l'anatoxine tétanique, et dans lequel le processus comprend les étapes consistant à combiner (i) un premier volume comprenant l'anatoxine diphtérique et l'anatoxine tétanique selon un ratio diphtérie : tétanos entre 2:1 et 3:1 (mesuré en unités Lf) avec (ii) un second volume comprenant l'anatoxine tétanique mais pas l'anatoxine diphtérique.

2. Processus selon la revendication 1, dans lequel le vaccin comprend aussi un ou plusieurs antigènes pertussiques acellulaires par exemple incluant la toxine pertussique inactivée, l'hémagglutinine filamenteuse et la pertactine.

3. Processus selon la revendication 2, dans lequel la toxine pertussique inactivée, l'hémagglutinine filamenteuse et la pertactine sont présentes selon un ratio de 8:8:3 (mesuré en poids).

4. Processus selon l'une quelconque des revendications précédentes, dans lequel le vaccin comprend aussi des antigènes inactivés du poliovirus (IPV), par exemple des antigènes IPV issus chacun d'une souche de poliovirus de type 1, d'une souche de poliovirus de type 2 et d'une souche de poliovirus de type 3.

5. Processus selon la revendication 4, dans lequel les antigènes IPV sont présents selon un ratio type 1:2:3 de 5:1:4 (mesuré par DU).

6. Processus selon l'une quelconque des revendications précédentes, dans lequel le vaccin comprend un ou plusieurs sels d'aluminium adjuvants, par exemple dans lequel le vaccin comprend un adjuvant hydroxyde d'aluminium et, facultativement, comprend en outre un adjuvant phosphate d'aluminium.

7. Processus selon la revendication 6, dans lequel le vaccin ne comprend pas d'adjuvant phosphate d'aluminium.

8. Processus selon l'une quelconque des revendications précédentes, dans lequel les anatoxines diphtériques et tétaniques dans le premier volume sont adsorbées sur un adjuvant hydroxyde d'aluminium.

9. Processus selon l'une quelconque des revendications précédentes, dans lequel le premier volume ne contient pas de conservateurs mercuriels.

10. Processus selon l'une quelconque des revendications précédentes, dans lequel le premier volume inclut un conservateur, par exemple 1-hydroxy-2-phénoxyéthane.

11. Processus selon la revendication 10, dans lequel le premier volume inclut environ 5 g/l de 1-hydroxy-2-phénoxyéthane.

12. Processus selon l'une quelconque des revendications 1 à 9, dans lequel le premier volume ne contient pas de conservateur.

13. Processus selon l'une quelconque des revendications précédentes, dans lequel l'anatoxine diphtérique et l'anatoxine tétanique dans le premier volume sont présentes à un ratio diphtérie:tétanos d'environ 2,5:1 (mesuré en unités Lf).

14. Processus selon l'une quelconque des revendications précédentes, dans lequel le premier volume inclut entre 5 mg et 6 mg de chlorure de sodium pour 100 Lf d'anatoxine diphtérique et/ou ne contient pas de polysorbate 80.

15. Processus selon l'une quelconque des revendications précédentes, dans lequel le second volume ne contient pas de sels d'aluminium et/ou ne contient pas de conservateurs mercuriels et/ou ne contient pas de conservateurs et/ou ne contient pas de polysorbate 80.

16. Processus selon l'une quelconque des revendications précédentes, dans lequel le vaccin a une teneur en Al³⁺ entre 0,25 mg/ml et 1,5 mg/ml, par exemple une teneur en Al³⁺ d'environ 0,6 mg/ml.

17. Processus selon l'une quelconque des revendications précédentes, comprenant en outre une étape consistant à emballer le vaccin dans des récipients, par exemple dans lequel le récipient est un flacon ou dans lequel le récipient est une seringue.

18. Processus selon l'une quelconque des revendications 2 à 17, dans lequel le vaccin a (i) la composition suivante par millilitre : 5 Lf d'anatoxine diphtérique ; 10 Lf d'anatoxine tétanique ; 16 µg de toxine pertussique inactivée ; 16 µg d'hémagglutinine filamenteuse ; 5 µg de pertactine ou (ii) la composition suivante par millilitre : 5 Lf d'anatoxine diphtérique ; 10 Lf d'anatoxine tétanique ; 16 µg de toxine pertussique inactivée ; 16 µg d'hémagglutinine filamenteuse ; 5 µg de pertactine ; 80 DU de poliovirus de souche Mahoney ; 16 DU de poliovirus de souche MEF1 et 64 DU de poliovirus souche Saukett.

19. Processus selon l'une quelconque des revendications précédentes, dans lequel le vaccin ne contient pas de conservateur.
